# EUROPEAN PATENT SPECIFICATION

(11) **EP 3 639 830 B1**
(45) Date of publication and mention of the grant of the patent: **21.08.2024**
(21) Application number: 18842959.1
(22) Date of filing: 20.07.2018
(51) Int. Cl.: A61K 33/36, A61L 31/10, A61L 31/16, A61P 9/10, A61P 7/02, A61K 9/00, A61K 31/436, A61K 31/285

(54) **COMPOSITE ANTI-RESTENOSIS DRUG FOR CORONARY DRUG-ELUTING STENT, AND CONTROLLED RELEASE SYSTEM THEREOF**
KOMPOSIT-ANTIRESTENOSE-ARZNEISTOFF FÜR MEDIKAMENTENBESCHICHTETEN KORONARSTENT UND GESTEUERTES FREISETZUNGSSYSTEM DAFÜR
MÉDICAMENT COMPOSITE ANTI-RESTÉNOSE POUR STENT CORONAIRE À ÉLUTION DE MÉDICAMENT ET SYSTÈME DE LIBÉRATION CONTRÔLÉE CORRESPONDANT

(30) Priority: 08.08.2017 CN 201710669568
(43) Date of publication of application: 22.04.2020
(73) Proprietor: Xinxin Medical Technology (Shanghai) Co. Ltd, Shanghai 201203 (CN)
(72) Inventor: HE, Fugui, Beijing 101500 (CN); MA, Xiaoyi, Beijing 101500 (CN); ZHANG, Li, Beijing 101500 (CN); ZHANG, Bili, Beijing 101500 (CN); YU, Bo, Beijing 101500 (CN); DOU, Kefei, Beijing 101500 (CN)
(74) Representative: Habermann, Hruschka & Schnabel
(86) International application number: PCT/CN2018/096393
(87) International publication number: WO 2019/029334

(56) References cited:
- CN-A- 104 707 185
- CN-A- 105 435 228
- CN-A- 106 073 942
- CN-A- 106 806 948
- CN-C- 100 435 756
- YANG W ET AL: "Arsenic trioxide eluting stent reduces neointima formation in a rabbit iliac artery injury model", CARDIOVASCULAR RESEARCH, OXFORD UNIVERSITY PRESS, GB, vol. 72, no. 3, 1 December 2006 (2006-12-01), pages 483 - 493, XP025011543, ISSN: 0008-6363, [retrieved on 20061201], DOI: 10.1016/J.CARDIORES.2006.08.010
- THIPPARABOINA: "Eluting combination drugs from stents", INTERNATIONAL JOURNAL OF PHARMACEUTICS, vol. 454, no. 1, September 2013 (2013-09-01), pages 4 - 10, XP055576454, ISSN: 0378-5173
- KONG HONGLIANG: "The Preventing Restenosis Effects of Rapamycin and Paclitaxel Eluting Stent ........", SHANXI MEDICAL JOURNAL, vol. 39, no. 4, 30 April 2010 (2010-04-30), pages 310 - 312, XP009518448

## Description

### Technical Field

The invention relates to a coronary drug-eluting stent for use in controlling restenosis and preventing thrombosis as defined in claim 1.

### Background

Coronary stent is an interventional medical device primarily used to treat ischemic heart disease due to coronary artery stenosis or occlusion, such as heart attack. Due to the inevitable damage to the coronary vessels during stent expansion, the vascular smooth muscle cells are hyperproliferative, resulting in postoperative in-stent restenosis (ISR). Early coronary stents are made of stainless steel, and a bare stent made of stainless steel has a restenosis rate of up to 20% or more.

With the development of stenting techniques, some anti-restenosis drugs are coated on the stent to form a drug-eluting stent, also known as a drug-releasing stent, in which the drug is mainly carried by a polymer coated on the surface of the metal stent. After the stent is placed in the intravascular lesion, the drug is controlled released upon elution from the polymer coating into the vessel wall tissues to exert a biomedical effect, preventing restenosis and thrombus in the stent after stent implantation. Currently, Sirolimus is the most widely used in-stent anti-restenosis drug since the discovery of drug stents, and Sirolimus derivatives are used by a few companies, such as Everolimus or Paclitaxel, which all show significant superiority against restenosis. In China, arsenic trioxide (As₂O₃) anti-restenosis drugs developed by Beijing AmsinoMed Medical Co., Ltd. have also achieved good results (CN101161300A). Due to the effect of anti-restenosis drugs, restenosis rate can be controlled to 5% or below.

However, long-term clinical observations of Sirolimus-eluting stents for more than a decade have shown that although the use of Sirolimus has greatly reduced in-stent restenosis, the incidence of intravascular thrombosis in patients has also increased. The main reason lies in that, while Sirolimus inhibits the hyperproliferation of vascular smooth muscles, it also indiscriminately inhibits the growth and repair of vascular endothelial cells. Because of the inhibitory effect of Sirolimus on endothelial cells, coronary vascular endothelium recovery is slow after implantation of a Sirolimus-eluting stent. Some clinical data showed that 10% of stents were not completely covered by endothelium 10 months after stent implantation, and 5% of patients developed fatal late thrombosis. It is well known that incomplete endothelialization after stent implantation is the main cause of intravascular thrombosis. Drug-eluting stents using Sirolimus increases the risk of intravascular thrombosis while significantly reducing in-stent restenosis. In order to prevent thrombosis, patients who use Sirolimus-eluting stents often need long-term or even life-long administration of antithrombotic drugs.

Currently, it is a great challenge in the development of stent interventional therapy to solve the problem of the inhibition of the growth and repair of vascular endothelial cells and subsequent thrombosis caused by anti-restenosis drugs.

Thipparabonia et al. (2013) International Journal of Pharmaceutics 454 (1), 4-10, report on the elution of drug combinations from stents.

CN 100 435 756 C discloses an arsenic trioxide-controlled release elution stent.

CN 104 707 185 A describes a ginkgolide B composite medicine eluting stent.

Yang et al. (2006) Cardiovascular Research 72 (3), 483-493, discloses an arsenic trioxide eluting stent.

### Summary of the Invention

The invention concerns a coronary drug-eluting stent for use in controlling restenosis and preventing thrombosis as defined in claim 1.Preferred embodiments are characterized in claims 2 to 10.

The inventors found in the study that a composite drug prepared from Sirolimus and arsenic trioxide in a certain ratio can reduce or prevent the inhibition of vascular endothelial cell growth and repair, or even accelerate the vascular endothelialization, while achieving a good anti-restenosis effect.

Although it has been reported in the prior art that both Sirolimus and arsenic trioxide can inhibit smooth muscle proliferation and act on the G1 phase of the cell growth cycle, the mechanism of action is different. Sirolimus is a novel macrolide-type immunosuppressive agent that blocks signaling through different cytokine receptors and blocks the progression from the G1 to S phase of T lymphocytes and other cells, thereby exhibiting an inhibitory effect ("The mechanism of action and clinical application of Sirolimus", Li Min, Zhao Ming, Department of Blood Purification and Kidney Transplantation, Zhujiang Hospital, First Military Medical University, Foreign Medicine and Pharmacy, June 2002, Vol. 29, No. 3). Mitochondria is the mostarsenic trioxide-sensitive organelle, and a previous study," Study on the Mechanisms of Arsenic Trioxide-induced Vascular Smooth Muscle Cells Apoptosis in vitro" (Li Hui et al., Journal of Medical Research, October 2010, Vol. 39, No. 10), found that As₂O₃ could induce a decrease of mitochondrial transmembrane potential (ΔΨm) in VSMCs, and the decrease of mitochondrial ΔΨm wassignificantly correlated to the apoptosis-inducing effect thereof. In the process of induced apoptosis, because the decrease of ΔΨm occurs earlier than morphological changes by apoptosis, it is a specific marker of apoptosis. Thus, it has been confirmed that arsenic trioxide induced a decrease of mitochondrial ΔΨm in VSMCs, which led to apoptosis in VSMCs. The use of Sirolimus in combination with arsenic trioxide as an anti-restenosis drug has not been reported in the prior art.

According to a specific embodiment of the present invention, in the composite drug for coronary drug-eluting stent for use according to the present invention, the weight ratio between Sirolimus and arsenic trioxide is 1:2 to 2:1.

According to the invention, in the composite drug for coronary drug-eluting stent for use according to the present invention, based on a stent having an opened external diameter of 3.0 mm, the amount of As₂O₃ used is 1 to 8 µg/mm and the amount of the Sirolimus used is 1 to 8 µg/mm.

According to a specific embodiment of the present invention, in the composite drug for coronary drug-eluting stent for use according to the present invention, a polymer material may be further combined, and the resultant composition may be used in the preparation of a composite drug controlled release system for coronary drug-eluting stent. Here, the polymer material may specifically include one or more of polyactide (PLA), Polyglycolide (PGA), Poly(lactic-co-glycolic acid) (PLGA), Polycaprolac(PCL),and Poly(3-hydroxybutyrate-co-3-hydroxyvalerate) (PHBV). The polymer material is primarily used as a drug carrier, and the ratio between the polymer material and the drug may be adjusted as necessary for the release of a specific drug. Preferably, the weight ratio between the polymer material and the drug used in the present invention is 1:0.3 to 1:10.

The present invention is based on experiments showing that arsenic trioxide at a certain concentration functions to promote endothelial cell proliferation to some extent. According to a specific embodiment of the present invention, in the composite drug for coronary drug eluting stent of the present invention, the use amount of Sirolimus which has an hindrance effect on endothelial recovery can be greatly reduced, and the amount of arsenic trioxide used can be increased, so as to accelerate the endothelialization process of blood vessels while achieving a good anti-restenosis effect. In addition, the composite drug for a coronary drug eluting stent according to the present invention acts on the coronary artery that is damaged upon stent implantation, and shows a stronger effect in inhibiting smooth muscle hyperproliferation and preventing in-stent restenosis than that by using a single drug.

The coronary drug-eluting stent for use in the invention comprises:
a drug layer formed by coating the composite drug for coronary drug-eluting stent for use according to the present invention on the stent.

According to a specific embodiment of the present invention, the coronary drug eluting stent for use in the present invention further comprises the above polymer material. The polymer material is used as a drug carrier and controls the release of the drug, which is preferably a degradable polymer material, for example, any one or more selected from polyactide, polyglycolide, poly(lactic-co-glycolic acid), polycaprolac, and poly(3-hydroxybutyrate-co-3-hydroxyvalerate). Preferably, the weight ratio between the degradable polymer material and the drug is between 1:0.3 to 1:10.

In the coronary drug eluting stent for use in the present invention, during the preparation of the composite drug for the coronary drug-eluting stent, As₂O₃ and Sirolimus, or As₂O₃, Sirolimus and the degradable polymer material may be mixed in a designed ratio (e.g., by simple mixing).

In the present invention, by further changing the structure (monolayer or multilayer) of the drug release system (drug coating) and the ratio between the drug and the polymer material in each layer, a controlled release of the drug may be realized, allowing the composite drug to release in accordance with the optimal profile and achieve the optimal therapeutic efficacy of both controlling restenosis and preventing thrombosis.

According to a specific embodiment of the present invention, in the coronary drug-eluting stent for use in the present invention, the drug layer is a composite drug monolayer structure; that is, a layer of the composite drug or the composite drug and degradable polymer material that are proportionally and uniformly mixed is included in the composite drug. In other words, the present invention makes use ofs a monolayer-structured composite drug release system. In this monolayer-structured composite drug release system, two anti-restenosis drugs (As₂O₃ and Sirolimus) are included, or a polymer material is further included. By adjusting the ratio between the drugs and that between the drugs and the polymer material, a change in the drug release profile is attained, thereby achieving the goal of controlled drug release. Upon consideration for further optimization, in the formulation of the composite drug, the amount of As₂O₃ used is 4 to 8 µg/mm, and the amount of the Sirolimus used is 1 to 5 µg/mm. These amounts are based on a stent having an opened external diameter of 3.0 mm, and the usage of drugs should be increased in proportion to the actual surface area of the stent for a stent having a larger opened external diameter. In such a structure, a layer of polymer material or other materials may be added between the single layer of the mixed drug layer and the stent surface, so as to increase the adhesion of the drug layer to the stent surface. A polymer material or other materials may be spray coated on the outer surface of the monolayer mixed drug layer in order to protect the mixed drug layer, preventing the mixed drug layer from rupture during stent implantation. It was observed in experiments that the release of Sirolimus did not change significantly upon mixing Sirolimus with arsenic trioxide but the releasing performance of arsenic trioxide was greatly improved. By appropriately adjusting the usage ratio between Sirolimus and arsenic trioxide, the release of both Sirolimus and arsenic trioxide may coincide with the release profile according to clinical requirements, to produce the optimal clinical results. Furthermore, since there is only one layer of mixed drug, the drug spraying process tothe stent becomes simple and easy. Due to the liposoluble nature of Sirolimus, it may readily penetrate the cell wall to exert the pro-apoptotic effect of the drug on cells. The molecular weight of Sirolimus is four times that of arsenic trioxide. It is quite likely that this mixed drug delivery system allows large molecules of Sirolimusto "carry" small molecules of arsenic trioxide into the cell to help arsenic trioxide to have a better pharmaceutical effect. In this structure, a layer of polymeric material or other materials may be added between the drug layer and the stent surface to increase the adhesion of the drug layer to the stent surface. A polymer material or other materials may be spray coated on the outer surface of the drug layer in order to protect the drug layer, preventing the drug layer from rupture during stent implantation. Preferably, in the monolayer-structured composite drug release system, a degradable polymer material of poly(lactic-co-glycolic acid) (PLGA) is used as a drug carrier.

According to another specific embodiment of the present invention, in the composite drug controlled release system used in the present invention, the drug layer is a composite drug multilayer structure; that is, a plurality of layers of the composite drug or the composite drug and degradable polymer material that are proportionally and uniformly mixed are included in the composite drug controlled release system. In the multilayer-structured controlled release system, each layer independently: comprises only one drug; comprises only the composite drug; comprises only the degradable polymer material; comprises only one drug and the degradable polymer material; comprises only the composite drug and the degradable polymer material. In other words, the present invention provides a multilayer-structured (each layer only comprising a single drug) composite drug release system. In this release system, there are a plurality of drug layers, with each layer comprising only a single drug and an optional polymer material, and at least one layer of arsenic trioxide drug layer and at least one layer of Sirolimus layer are included in the overall structure. By adjusting the number of drug layers, the ratio between the drugs and the polymer material in each layer and the relative position of each drug layer, an optimal drug release is achieved. With regard to the drug usage, the total amount of As₂O₃ used in the overall system is preferably 4 to 8 µg/mm and the total amount of the Sirolimus used in the overall system is preferably 1 to 5 µg/mm. These amounts are based on a stent having an opened external diameter of 3.0 mm, and the usage of drugs should be increased in proportion to the actual surface area of the stent for a stent having a larger opened external diameter. In such a structure, a layer of polymer material or other materials may be added between the lowest drug layer and the stent surface, so as to increase the adhesion of the drug layer to the stent surface. A polymer material or other materials may be spray coated on the outer surface of the outermost drug layer in order to protect the drug layers, preventing the drug layers from rupture during stent implantation. Preferably, in the monolayer-structured composite drug release system, a degradable polymer material of poly(lactic-co-glycolic acid) (PLGA) is used as a drug carrier.

According to still another specific embodiment of the present invention, the coronary drug-eluting stent for use according to the present invention includes a multilayer-structured (each layer comprising a mixed drug of one or two or more drugs) composite drug release system. In this release system, there are a plurality of drug layers, with each layer comprising one or two or more drugs and an optional polymer material. By adjusting the number of drug layers, the ratio between the drugs and that between the drugs and the polymer material in each layer, as well as the relative position of each drug layer, an optimal drug release is achieved. With regard to the drug usage, the total amount of As₂O₃ used in the overall system is preferably 2 to 10µg/mm and the total amount of the Sirolimus used in the overall system is preferably 2 to 12µg/mm. These amounts are based on a stent having an opened external diameter of 3.0 mm, and the usage of drugs should be increased in proportion to the actual surface area of the stent for a stent having a larger opened external diameter. In such a structure, a layer of polymer material or other materials may be added between the lowest drug layer and the stent surface, so as to increase the adhesion of the drug layer to the stent surface. A polymer material or other materials may be spray coated on the outer surface of the outermost drug layer in order to protect the drug layers, preventing the drug layers from rupture during stent implantation.

The present invention has the following technical effects:
The composite drug for coronary drug-eluting stent for use according to the present invention has an inhibitory effect on smooth muscle cell proliferation and, due to the reduced amount of Sirolimus used, can reduce the damage of the drug to endothelial cells and further effect to facilitate endothelial cell recovery, thereby achieving excellent in-stent anti-restenosis effect after stent implantation and lowering the incidence of intravascular thrombosis. The composite drug controlled release system of the coronary drug-eluting stent for use according to the present invention may allow the composite drug to release in accordance with an optimal profile and achieve the optimal therapeutic efficacy of both controlling restenosis and preventing thrombosis.

Sirolimus has been used for an anti-restenosis drug stent for a long time, and its pro-apoptotic effect on smooth muscle cells and endothelial cells in coronary vessels has been substantiated in tremendous studies and in clinic. As for arsenic trioxide, although it has been used as a drug for treating leukemia for a long time, there still lacks investigation of the mechanism of its *in vivo* and *in vitro* action on cells when used in a drug stent. In the present invention, in vitro experiments with arsenic trioxide on smooth muscle cells and endothelial cells of porcine coronary vessels are performed, and a series of significant experimental results are observed. These results confirm that arsenic trioxide has a strong pro-apoptotic effect on smooth muscle cells when it reaches a certain dose. However, for endothelial cells, the pro-apoptotic effect of arsenic trioxide is not obvious. On the contrary, on the first to the third day upon drug action, there even shows a stimulation of growth. This effect is completely different from the simple pro-apoptotic effect of Sirolimus on endothelial cells, and may have a positive impact on rapid endothelial repair.

The present invention also provides two configurations for the controlled release system of a composite drug of As₂O₃ and Sirolimus in the coronary drug -eluting stent of use according to the invention: a monolayer-structured composite drug release system and a multilayer-structured (each layer comprising one drug or a mixture of one or two or more drugs) composite drug release system. It is found in the experiments on these controlled release systems that Sirolimus may accelerate the release of As₂O₃ from polymer materials, and further by intricately designing the controlled release system, it is possible for both As₂O₃ and Sirolimus to achieve optimal release and good clinical results in a controlled release system while substantially reducing the amount of Sirolimus used.

### Brief Description of the Accompanying Drawings

Figure 1 shows the effect of arsenic trioxide on primary porcine coronary vascular smooth muscle cells in an in vitro experiment.
Figure 2 shows the effect of arsenic trioxide on primary porcine coronary endothelial cells in an in vitro experiment.
Figure 3 shows the profile of the arsenic trioxide release rate of a drug coating in which the mixing ratio of arsenic trioxide to polymer material (degradable PLGA) is 1:1.
Figure 4 shows the profile of the arsenic trioxide release rate of a drug coating in which the mixing ratio between arsenic trioxide, polymer material (degradable PLGA) and Sirolimus is 1: 1: 0.4.
Figure 5A shows the OCT results of porcine coronary (LAD) lumen and vascular endothelium repair when a stent with optimized composite drug (Group 4, monolayer structure, Formulation B) is used. In the figure, the bright short-bar image along the inner wall of the vessel is the cross section of the stent.
Figure 5B shows the OCT angiography of pure arsenic trioxide-plated stent (3.0 x 17 mm, control group 1, monolayer structure) one month after being implanted into the porcine right circumflex coronary artery.
Figure 5C shows the OCT angiography of a pure RAPA-plated stent (3.0 x 17 mm, control group 2, monolayer structure)one month after being implanted into the porcine left circumflex coronary artery.
Figure 5D shows the OCT angiography of a composite drug stent (3.0 x 17 mm) with the composite drug monolayer-structured release system (Implementation Method 1, Group 3, monolayer structure, Formulation A) three months after being implanted into the porcine left circumflex coronary artery.
Figure 5E shows the OCT angiography of a composite drug stent (3.0 x 17 mm) with the composite drug multilayer-structured release system (each layer comprising only a single drug of arsenic trioxide or Sirolimus, Implementation Method 2, Group 5, bilayer structure) composite drug stent (3.0 x 17 mm) three months after being implanted into the porcine left anterior descending coronary artery.
Figure 6 shows a profile of optimized arsenic trioxide cumulative release.
Figure 7 shows a profile of Sirolimus cumulative release in the controlled release system of Group 4.

### Detailed Description of the Invention

For better understanding of the technical features, purposes and beneficial effects of the present invention, the technical solutions of the present invention will now be set forth in details in connection with the specific examples and the accompanying drawings. It will be appreciated that these examples are merely illustrative of the present invention and are not intended to limit the scope of the present invention.

### Example 1

### 1. In vitro experiment of the effect of arsenic trioxide on smooth muscle cells and endothelial cells of porcine coronary vessels

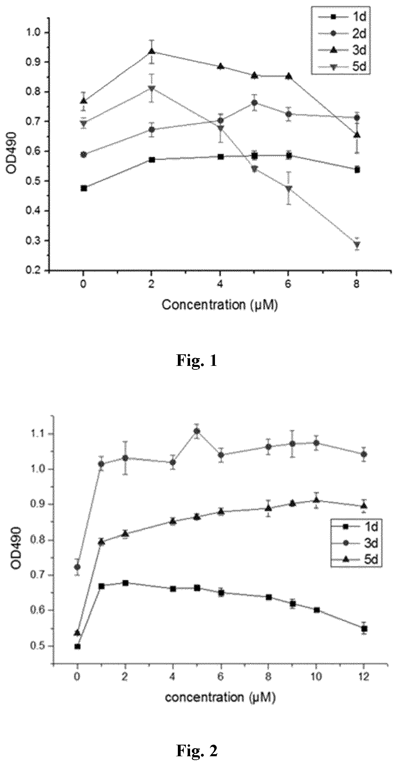

Figures 1 and 2 show the effects of arsenic trioxide on primary smooth muscle cells and primary endothelial cells of porcine coronary vessels in in vitro experiments. In the figures, the vertical axis represents the total number of cells in the wells of the culture plate, and the horizontal axis represents the concentration of the arsenic trioxide solution. Each curve represents the effect of arsenic trioxide at various concentrations on apoptosis (decrease in total number of cells) at a given time. It can be clearly seen from the figure (Fig. 2) that arsenic trioxide has little effect of on endothelial cells (at a concentration of 12 µM or below) and even has an effect of promoting proliferation of endothelial cell to a certain extent (Fig. 2). However, for smooth muscle cells, cells apoptosis began even at low concentrations (3 µM) from Day 2 (Fig. 1). It is sufficiently demonstrated that arsenic trioxide has different effects on different types of cells of coronary vessels. Arsenic trioxide may induce apoptosis of smooth muscle cells while having poor inhibitory effect on endothelial cells or even promoting their growth.

### 2. Impact of adding Sirolimusin the drug delivery system on arsenic trioxide release

In designing a drug controlled release system for a drug-eluting stent, the drug and degradable polymer material must be mixed together in a certain manner to be sprayed on the stent, and the drug will be released from the polymer materials after the stent is implanted. Sirolimus can be dissolved in a variety of common organic solvents, and controlled release system can be easily designed by using Sirolimus as an anti-restenosis drug. Arsenic trioxide is an inorganic oxide, with only one crystal form thereof out of three slightly soluble in water, while it cannot be dissolved in common solvents such as ethanol. This makes the subsequent mixing with the polymer material difficult. In addition, it has shown in the experiments that even if arsenic trioxide was mixed in a polymer material in a particulate state, the release rate of the drug could not meet the requirement for clinically effective therapeutic effect until the arsenic trioxide reached a very high concentration. However, this high concentration state significantly reduces the total spraying amount (0.3 µg/mm²) of the polymer material and the drug, which not only makes it difficult to control the spraying process but affects the uniformity of the drug in clinical use as well.

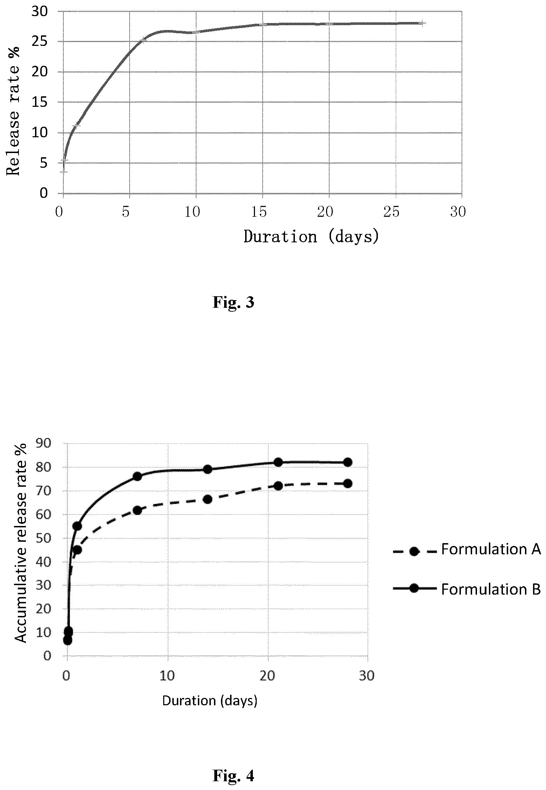

Figure 3 shows profile of arsenic trioxide release from a drug coating in which the mixing ratio of arsenic trioxide/polymer material is 1:1. The release rate reached a maximum of about 28% in about a week and then increased very slowly.

After adding Sirolimus to the arsenic trioxide/polymer material mixture at the same ratio (Fig. 4, the mixed ratio of arsenic trioxide, polymer material and Sirolimus is 1: 1: 0.4), for the stents of all sizes in the experiment, The release rate of arsenic trioxide was almost three times that of the original.

### Example 2

In this example, two implementation methods, which achieve controlled drug release by changing the structure (monolayer or multilayer) of the drug release system (drug coating) and the ratio between drug and polymer material in each layer, are exemplified.

### Implementation method 1: monolayer-structured composite drug release system

In this monolayer-structured release system of mixed drugs, arsenic trioxide, Sirolimus, and a polymer material PLGA are included. By adjusting the ratio between drugs and that between the drug and the polymer material, the drug release profile is changed to achieve the objective of controlled drug release.

As long as there is no adverse interaction between the mixed drugs, a variety of anti-restenosis drugs such as arsenic trioxide, Sirolimus, paclitaxel or the like can be selected for drug mixing in theory. In consideration of clinical and manufacturing optimization, two drugs, arsenic trioxide and Sirolimus, may be selected for mixing. Upon consideration for further optimization, in the formulation of the composite drug, the amount of As₂O₃ used is 4 to 8 µg/mm, and the amount of the Sirolimus used is 1 to 5 µg/mm. These amounts are based on a stent having an opened external diameter of 3.0 mm, and the use amount of drugs should be increased in proportion to the actual surface area of the stent for a stent having a larger opened external diameter.

In such a structure, a layer of polymer material or other materials may be added between the single layer of the mixed drug layer and the stent surface, so as to increase the adhesion of the drug layer to the stent surface. A polymer material or other materials may be spray coated on the outer surface of the monolayer mixed drug layer in order to protect the mixed drug layer, preventing the mixed drug layer from rupture during stent implantation.

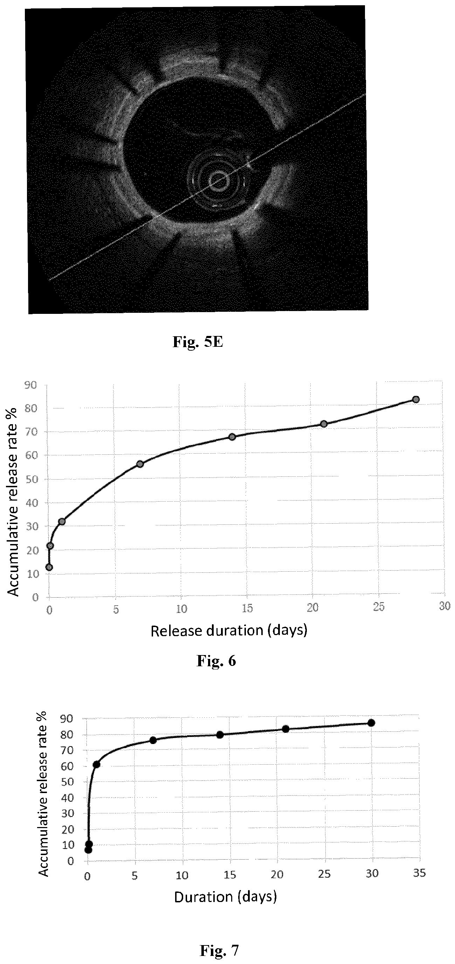

In the process of implementing the monolayer-structured composite drug release system, the ratio between Sirolimus and the degradable polymer material PLGA could be adjusted to allow the release of Sirolimus to reach an optimal profile (Fig. 6), and arsenic trioxide was further added into the system without any substantial impact on the Sirolimus release profile. However, as the mixed ratio between arsenic trioxide and Sirolimus varied, the release rate of arsenic trioxide also changed. The release rate of arsenic trioxide increased as the ratio of arsenic trioxide to Sirolimus became higher. Fig. 4 shows these changes, in which the ratio of arsenic trioxide to Sirolimus is 5:2 in Formulation B and the ratio of arsenic trioxide to Sirolimus is 5:4 in Formulation A, with the release rate of arsenic trioxide of Formulation B significantly higher than that of Formulation A, which is substantially consistent with the optimal profile of arsenic trioxide release (Fig. 7). The stent with Formulation B also performed better in subsequent animal model tests.

With the mixing of Sirolimus and arsenic trioxide, the release property of arsenic trioxide is greatly improved, and it is thus possible to make the release of both Sirolimus and arsenic trioxide meet the release profile in accordance with clinical requirements by properly adjusting the usage ratio of Sirolimus to arsenic trioxide, thereby producing optimal clinical results. Also, since there is only one mixed drug layer, the drug spraying process for the stent becomes simple and easy.

### Implementation method 2: multilayer-structured (each layer comprising a single drug) composite drug release system

In this release system, there are a plurality of drug layers, with each layer comprising only a single drug and an optional polymer material. By adjusting the number of drug layers, the ratio between the drugs and the polymer material in each layer and the relative position of each drug layer, an optimal drug release is achieved.

With regard to the use amount for the drug, the total amount of As₂O₃ used in the overall system is 4 to 8 µg/mm and the total amount of the Sirolimus used in the overall system is 1 to 5 µg/mm. These amounts are based on a stent having an opened external diameter of 3.0 mm, and the use amount of drugs should be increased in proportion to the actual surface area of the stent for a stent having a larger opened external diameter.

In such a structure, a layer of polymer material or other materials with good biocompatibility may be added between the lowest drug layer and the stent surface, for example, a layer of porous metal titanium may be plated on the metallic stent surface, so as to increase the adhesion of the drug layer to the stent surface. A polymer material or other materials may be spray coated on the outer surface of the outermost drug layer in order to protect the drug layers, preventing the drug layers from rupture during stent implantation.

### Implementation method 3: multilayer-structured (each layer comprising one drug or a mixed drug of two or more drugs) composite drug release system

In this release system, there are a plurality of drug layers, with each layer comprising one or two or more drugs and an optional polymer material. By adjusting the number of drug layers, the ratio between the drugs and that between the drugs and the polymer material in each layer, as well as the relative position of each drug layer, an optimal drug release is achieved.

With regard to the drug use amount, the total amount of As₂O₃ used in the overall system is 2 to 10 µg/mm and the total amount of the Sirolimus used in the overall system is 2 to 12 µg/mm. These amounts are based on a stent having an opened external diameter of 3.0 mm, and the use amount of drugs should be increased in proportion to the actual surface area of the stent for a stent having a larger opened external diameter.

In such a structure, a layer of polymer material or other materials with good biocompatibility may be added between the lowest drug layer and the stent surface, so as to increase the adhesion of the drug layer to the stent surface. A polymer material or other materials may be spray coated on the outer surface of the outermost drug layer in order to protect the drug layers, preventing the drug layers from rupture during stent implantation.

### Animal model experiment results with implementation methods 1 and 2

Comparative experiments were conducted on porcine coronary animal models by using drug-eluting stents prepared by the implementation methods 1 and 2, respectively. Grouping is shown below:

| **Group** | **Drug** | **Implementation method** | **Structure of the coating layer** | **Number of stents implanted** |
|---|---|---|---|---|
| 1 | As₂O₃ | Control group | Monolayer structure | 7 |
| | | | As₂O₃ : 3µg/mm + PLGA surface protecting layer | |
| 2 | Sirolimus | Control group | Monolayer structure | 8 |
| | | | RAPA: 8µg/mm | |
| | | | PLGA: 18µg/mm | |
| 3 | As₂O₃ + Sirolimus | Method 1 | Mixed drug monolayer structure (Formulation A) | 10 |
| | | | As₂O₃:5µg/mm | |
| | | | RAPA: 4µg/mm | |
| | | | PLGA: 9µg/mm | |
| 4 | As₂O₃ + Sirolimus | Method 1 | Mixed drug monolayer structure (Formulation B) | 8 |
| | | | As₂O₃:5µg/mm | |
| | | | RAPA: 2µg/mm | |
| | | | PLGA: 4.6 µg/mm | |
| 5 | As₂O₃ + Sirolimus | Method 2 | Bilayered drug structure | 10 |
| | | | As₂O₃:3µg/mm | |
| | | | RAPA: 4µg/mm +PLGA surface protecting layer | |

Coronary angiography and OCT examination results from some stents afterstent implantation are shown in the following table:

| **Group** | **Drug** | **Implementation method** | **Number of stents implanted** | **Duration of implantation** | **Examination method** | **Result** |
|---|---|---|---|---|---|---|
| 1 | As₂O₃ | Control group | 7 | 1 month | Angiography | 3 stents with mediate to mild stenosis The rest fine |
| 2 | Sirolimus | Control group | 8 | 1 month | Angiography | 3 stents with mediate to mild stenosis The rest fine |
| 3 | As₂O₃ + Sirolimus | Method 1 | 10 | 3 months | Angiography, OCT | All showed good angiography, with no in-stent restenosis; 2 stents with mild stenosis at the opening 3 were subjected to OCT observation Good endothelialization |
| 4 | As₂O₃ + Sirolimus | Method 1 | 8 | 3 months | Angiography, OCT | All showed good angiography, with no in-stent restenosis; 3 stents with mild stenosis at the opening 3 were subjected to OCT observation Good endothelialization |
| 5 | As₂O₃ + Sirolimus | Method 2 | 10 | 3 months | Angiography,OCT | All showed good angiography, with no in-stent restenosis; 4 stents with mild stenosis at the opening 4 were subjected to OCT observation Good endothelialization, with 2 having thicker endothelium |

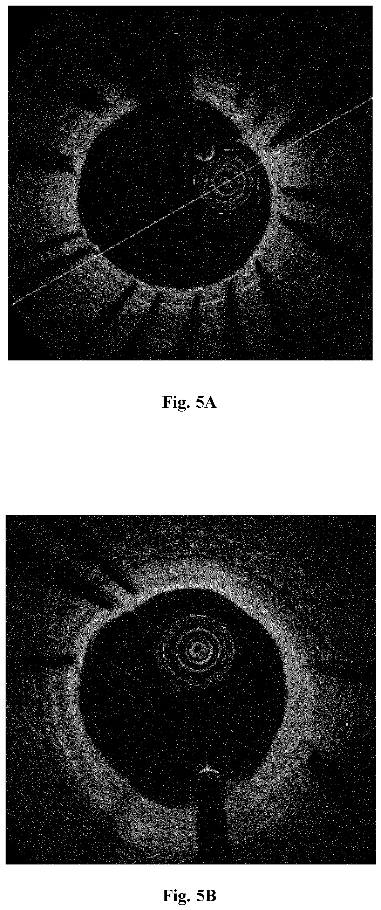

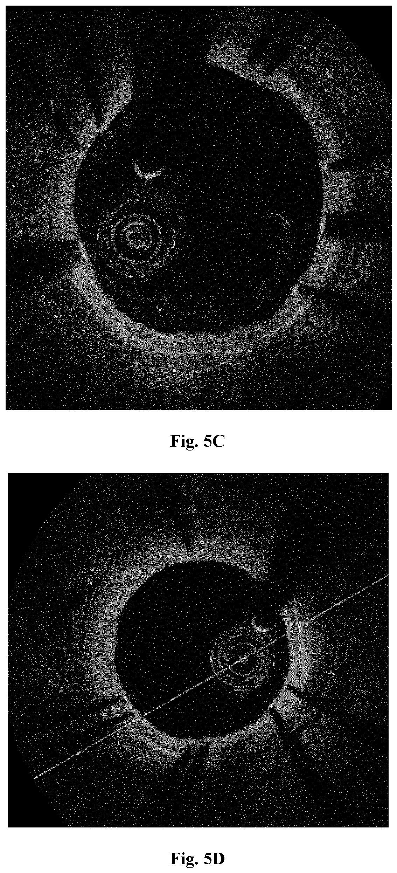

Groups 1 and 2 were stents with pure arsenic trioxide or Sirolimus respectively that were used as a control group. Groups 3, 4, and 5 were stents with a composite drug (arsenic trioxide and Sirolimus). As compared to those with pure anti-restenosis drugs (arsenic trioxide or Sirolimusas used in this experiment), stents with the composite drug (arsenic trioxide and Sirolimus) showed better in-stent anti-restenosis ability under coronary angiography conditions. The results from OCT observation showed that the coronary vascular endothelium completely covered the stent surface within 3 months when the stent with composite drug was used. Here, Figure 5A shows the OCT results of porcine coronary (LAD) lumen and vascular endothelium repair when a stent with optimized composite drug (Group 4, monolayer structure, Formulation B)was used; in this figure, the bright short-bar image along the inner wall of the vessel is the cross section of the stent, showing that no in-stent restenosis is observed and the stent is completely covered by the endothelium three months after the stent with the monolayer mixed drug formulation B (Group 4) was implanted. Figure 5B shows the OCT angiography of pure arsenic trioxide-plated stent (3.0 x 17 mm, control group 1, monolayer structure) one month after being implanted into the porcine right circumflex coronary artery; the image shows that the stent surface is completely covered by the endothelium, with a slightly thicker endothelium. Figure 5C shows the OCT angiography of a pure RAPA-plated stent (3.0 x 17 mm, control group 2, monolayer structure)one month after being implanted into the porcine left circumflex coronary artery; the image shows that part of the stent surface is not well covered by the endothelium. Figure 5D shows the OCT angiography of a composite drug stent (3.0 x 17 mm) with the composite drug monolayer-structured release system (Implementation Method 1, Group 3, monolayer structure, Formulation A) three months after being implanted into the porcine left circumflex coronary artery; the image shows that the stent surface is completely covered by the endothelium with an uniform endothelial thickness. Figure 5E shows the OCT angiography of a composite drug stent (3.0 x 17 mm) with the composite drug multilayer-structured release system (each layer comprising only a single drug of arsenic trioxide or Sirolimus, Implementation Method 2, Group 5, bilayer structure) composite drug stent (3.0 x 17 mm) three months after being implanted into the porcine left anterior descending coronary artery; the image shows that the stent surface is completely covered by the endothelium with an uniform endothelial thickness.

Last but not least, it should be noted that the above examples are merely illustrative of the implementation processes and features of the present invention and not limiting the technical solutions of the present invention. Even though the present invention are described in details with reference to these examples, persons skilled in the art would recognize that modification or equivalent substitution can be made to the present invention.

## Claims

1. A coronary drug-eluting stent for use in controlling restenosis and preventing thrombosis, comprising a stent and a controlled release drug layer formed by coating a composite drug on the stent, wherein the composite drug comprises As₂O₃ and Sirolimus, and based on a stent having an opened external diameter of 3.0 mm, the amount of As₂O₃ used is 1 to 8 µg/mm and the amount of the Sirolimus used is 1 to 8 µg/mm.

2. The coronary drug-eluting stent for use in controlling restenosis and preventing thrombosis as claimed in claim 1, further comprising a degradable polymer material as a drug carrier to control the drug release, which degradable polymer material includes one or more of polylactide (PLA), polyglycolide (PGA), poly(lactic-co-glycolic acid) (PLGA), polycaprolac(PCL),and poly(3-hydroxybutyrate-co-3-hydroxyvalerate) (PHBV).

3. The coronary drug-eluting stent for use in controlling restenosis and preventing thrombosis as claimed in claim 2, wherein the weight ratio between the degradable polymer material and the drug is between 1:0.3 to 1:10.

4. The coronary drug-eluting stent for use in controlling restenosis and preventing thrombosis as claimed in claims 1 to 3, wherein As₂O₃ and Sirolimus, or As₂O₃, Sirolimus and the degradable polymer material are mixed.

5. The coronary drug-eluting stent for use in controlling restenosis and preventing thrombosis as claimed in any one of claims 1 to 4, wherein the drug layer is a composite drug monolayer structure, that is, a layer of the composite drug or the composite drug and the degradable polymer material that are uniformly mixed is included in the drug layer.

6. The coronary drug-eluting stent for use in controlling restenosis and preventing thrombosis as claimed in claim 5, wherein, based on the stent having an opened external diameter of 3.0 mm, the amount of As₂O₃ used is 4 to 8 µg/mm and the amount of the Sirolimus used is 1 to 5 µg/mm.

7. The coronary drug-eluting stent for use in controlling restenosis and preventing thrombosis as claimed in any one of claims 1 to 4, wherein the drug layer is a composite drug multilayer structure, that is, a plurality of layers of the composite drug or the composite drug and the degradable polymer material that are uniformly mixed are included in the drug layer, wherein in the multilayer-structured drug layer, each layer independently:
comprises only one drug;
comprises only the composite drug;
comprises only the degradable polymer material;
comprises only one drug and the degradable polymer material;
comprises only the composite drug and the degradable polymer material.

8. The coronary drug-eluting stent for use in controlling restenosis and preventing thrombosis as claimed in claim 7, wherein, based on the stent having an opened external diameter of 3.0 mm, the total amount of As₂O₃ used in the drug layer is 4 to 8 µg/mm and the total amount of the Sirolimus used in the drug layer is 1 to 5 µg/mm.

9. The coronary drug-eluting stent for use in controlling restenosis and preventing thrombosis as claimed in any one of claims 2 to 7, further comprising a transitional layer interposed between the stent surface and the drug layer having a material selected from a degradable polymer material or other materials with good biocompatibility.

10. The coronary drug-eluting stent for use in controlling restenosis and preventing thrombosis as claimed in claim 9, further comprising a surface protecting layer consisted of a degradable polymer material.

## Patentansprüche

1. Koronararzneimittelfreisetzender Stent zur Verwendung in der Bekämpfung einer Restenose und der Verhinderung von Thrombose, umfassend einen Stent und eine Arzneimittelschicht mit kontrollierter Freisetzung, die durch Beschichtung eines Verbundarzneimittels auf dem Stent gebildet wird, wobei das Verbundarzneimittel As₂O₃ und Sirolimus umfasst und, basierend auf eines Stents mit einem geöffneten Außendurchmesser von 3,0 mm, die Menge des verwendeten As₂O₃ 1 bis 8 µg/mm und die Menge des verwendeten Sirolimus 1 bis 8 µg/mm beträgt.

2. Koronararzneimittelfreisetzender Stent zur Verwendung in der Bekämpfung von Restenose und der Verhinderung von Thrombose nach Anspruch 1, ferner umfassend ein abbaubares Polymermaterial als Arzneimittelträger zur Kontrolle der Arzneimittelfreisetzung, wobei das abbaubare Polymermaterial eines oder mehrere von Polylactid (PLA), Polyglycolid (PGA), Poly(lactid-co-glycolsäure) (PLGA), Polycaprolacton (PCL) und Poly(3-hydroxybutyrat-co-3-hydroxyvalerat) (PHBV) einschließt.

3. Koronararzneimittelfreisetzender Stent zur Verwendung in der Bekämpfung von Restenose und der Verhinderung von Thrombose nach Anspruch 2, wobei das Gewichtsverhältnis zwischen dem abbaubaren Polymermaterial und dem Arzneimittel zwischen 1:0,3 und 1:10 beträgt.

4. Koronararzneimittelfreisetzender Stent zur Verwendung in der Bekämpfung von Restenose und der Verhinderung von Thrombose nach einem der Ansprüche 1 bis 3, wobei As₂O₃ und Sirolimus oder As₂O₃, Sirolimus und das abbaubare Polymermaterial gemischt sind.

5. Koronararzneimittelfreisetzender Stent zur Verwendung in der Bekämpfung von Restenose und der Verhinderung von Thrombose nach einem der Ansprüche 1 bis 4, wobei die Arzneimittelschicht eine Verbundarzneimittel-Monolayer-Struktur ist, d.h. dass eine Schicht des Verbundarzneimittels oder des Verbundarzneimittels und des abbaubaren Polymermaterials, die gleichmäßig gemischt sind, in der Arzneimittelschicht enthalten ist.

6. Koronararzneimittelfreisetzender Stent zur Verwendung in der Bekämpfung von Restenose und der Verhinderung von Thrombose nach Anspruch 5, wobei, basierend auf dem Stent mit einem geöffneten Außendurchmesser von 3,0 mm, die Menge des verwendeten As₂O₃ 4 bis 8 µg/mm und die Menge des verwendeten Sirolimus 1 bis 5 µg/mm beträgt.

7. Koronararzneimittelfreisetzender Stent zur Verwendung in der Bekämpfung von Restenose und der Verhinderung von Thrombose nach einem der Ansprüche 1 bis 4, wobei die Arzneimittelschicht eine Verbundarzneimittel-Mehrschichtstruktur ist, d.h. eine Vielzahl von Schichten des Verbundarzneimittels oder des Verbundarzneimittels und des abbaubaren Polymermaterials, die gleichmäßig gemischt sind, in der Arzneimittelschicht enthalten sind, wobei in der Arzneimittelschicht mit Mehrschichtstruktur jede Schicht unabhängig voneinander:
nur ein Arzneimittel umfasst;
nur das Verbundarzneimittel umfasst;
nur das abbaubare Polymermaterial umfasst;
nur das Arzneimittel und das abbaubaren Polymermaterial umfasst;
nur das Verbundarzneimittel und das abbaubare Polymermaterial umfasst.

8. Koronararzneimittelfreisetzender Stent zur Verwendung in der Bekämpfung von Restenose und der Verhinderung von Thrombose nach Anspruch 7, wobei, basierend auf dem Stent mit einem geöffneten Außendurchmesser von 3,0 mm, die Gesamtmenge des in der Arzneimittelschicht verwendeten As₂O₃ 4 bis 8 µg/mm und die Gesamtmenge des in der Arzneimittelschicht verwendeten Sirolimus 1 bis 5 µg/mm beträgt.

9. Koronararzneimittelfreisetzender Stent zur Verwendung in der Bekämpfung von Restenose und der Verhinderung von Thrombose nach einem der Ansprüche 2 bis 7, ferner umfassend eine zwischen der Stentoberfläche und der Arzneimittelschicht angeordnete Übergangsschicht mit einem Material, das aus einem abbaubaren Polymermaterial oder anderen Materialien mit guter Biokompatibilität ausgewählt ist.

10. Koronararzneimittelfreisetzender Stent zur Verwendung in der Bekämpfung von Restenose und der Verhinderung von Thrombose nach Anspruch 9, der ferner eine Oberflächenschutzschicht aus einem abbaubaren Polymermaterial umfasst.

## Revendications

1. Un stent coronaire à élution de médicaments à utiliser pour contrôler la resténose et prévenir la thrombose, comprenant un stent et une couche de médicament à libération contrôlée formée par l'enrobage d'un médicament composite sur le stent, dans lequel le médicament composite comprend de l' As₂O₃ et du Sirolimus, et sur la base d'un stent ayant un diamètre externe ouvert de 3,0 mm, la quantité d' As₂O₃ utilisée est de 1 à 8 µg/mm et la quantité de Sirolimus utilisée est de 1 à 8 µg/mm.

2. Le stent coronaire à élution de médicaments à utiliser pour contrôler la resténose et prévenir la thrombose selon la revendication 1, comprenant en outre un matériau polymère dégradable comme vecteur de médicament pour contrôler la libération du médicament, ce matériau polymère dégradable comprenant un ou plusieurs des matériaux suivants : polylactide (PLA), polyglycolide (PGA), poly(acide lactique-co-glycolique) (PLGA), polycaprolac (PCL), et poly(3-hydroxybutyrate-co-3-hydroxyvalérate) (PHBV).

3. Le stent coronaire à élution de médicaments à utiliser pour contrôler la resténose et prévenir la thrombose selon la revendication 2, dans lequel le rapport de poids entre le matériau polymère dégradable et le médicament est compris entre 1:0,3 et 1:10.

4. Le stent coronaire à élution de médicaments utilisé pour contrôler la resténose et prévenir la thrombose selon les revendications 1 à 3, dans lequel l' As₂O₃ et le Sirolimus, ou l' As₂O₃, le Sirolimus et le matériau polymère dégradable sont mélangés.

5. Le stent coronaire à élution de médicaments à utiliser pour contrôler la resténose et prévenir la thrombose selon l'une des revendications 1 à 4, dans laquelle la couche de médicament est une structure monocouche de médicament composite, c'est-à-dire qu'une couche de médicament composite ou de médicament composite et de matériau polymère dégradable uniformément mélangés est incluse dans la couche de médicament.

6. Le stent coronaire à élution de médicaments à utiliser pour contrôler la resténose et prévenir la thrombose selon la revendication 5, dans lequel, sur la base du stent ayant un diamètre externe ouvert de 3,0 mm, la quantité d' As₂O₃ utilisée est de 4 à 8 µg/mm et la quantité de Sirolimus utilisée est de 1 à 5 µg/mm.

7. Le stent coronaire à élution de médicaments à utiliser pour contrôler la resténose et prévenir la thrombose selon l'une des revendications 1 à 4, dans laquelle la couche de médicament est une structure multicouche de médicament composite, c'est-à-dire qu'une pluralité de couches du médicament composite ou du médicament composite et du matériau polymère dégradable qui sont uniformément mélangées sont incluses dans la couche de médicament, où dans la couche de médicament à structure multicouche, chaque couche est indépendante :
ne comprend qu'un seul médicament ;
ne comprend que le médicament composite ;
ne comprend que le matériau polymère dégradable ;
ne comprend qu'un seul médicament et un matériau polymère dégradable ;
ne comprend que le médicament composite et le matériau polymère dégradable.

8. Le stent coronaire à élution de médicaments à utiliser pour contrôler la resténose et prévenir la thrombose selon la revendication 7, dans lequel, sur la base du stent ayant un diamètre externe ouvert de 3,0 mm, la quantité totale d' As₂O₃ utilisée dans la couche médicamenteuse est de 4 à 8 µg/mm et la quantité totale de Sirolimus utilisée dans la couche médicamenteuse est de 1 à 5 µg/mm.

9. Le stent coronaire à élution de médicaments à utiliser pour contrôler la resténose et prévenir la thrombose selon l'une des revendications 2 à 7, comprenant en outre une couche de transition interposée entre la surface de l'endoprothèse et la couche de médicament, constituée d'un matériau choisi parmi un polymère dégradable ou d'autres matériaux présentant une bonne biocompatibilité.

10. Le stent coronaire à élution de médicaments à utiliser pour contrôler la resténose et prévenir la thrombose selon la revendication 9, comprenant en outre une couche de protection de surface constituée d'un matériau polymère dégradable.
